# EUROPEAN PATENT APPLICATION

(11) **EP 4 579 683 A2**
(43) Date of publication of application: **02.07.2025**
(21) Application number: 25176902.2
(22) Date of filing: 09.08.2022
(51) Int. Cl.: G16H 50/30

(54) **IMAGE-BASED DEMENTIA DIAGNOSIS SYSTEM AND METHOD**

(30) Priority: 09.08.2021 KR 20210104505; 09.08.2021 KR 20210104512
(62) Divisional of application: 22856156.9
(71) Applicant: Emocog Inc., Seoul 08708 (KR)
(72) Inventor: LEE, Jun Young, 04427 Seoul (KR); NOH, Yoo Hun, 06346 Seoul (KR)
(74) Representative: Zacco Sweden AB

(57) **Abstract**

The present disclosure relates to an image-based dementia diagnosis system which enables a dementia diagnosis to be quickly and conveniently performed without using professional manpower, an early diagnosis of dementia and selection of a mild patient to be quickly and accurately performed, and even an elderly subject who is unable to recognize text to easily perform a checkup by presenting an image, wherein the dementia diagnosis system includes a text input device (10) through which a test subject inputs text, a data storage device (20) storing data for a diagnosis test, a display (30) configured to display various types of images and data, a selection manipulator (40) manipulated by the test subject to select an item, and controller (50) connected to various devices including the text input device (10), controlling the devices, and determining dementia by testing a memory on the basis of ani mage.

## Description

### Technical Field

The present disclosure relates to a dementia diagnosis system and method, and more particularly, to an image-based dementia diagnosis system and method which enable dementia diagnosis to be quickly and conveniently performed without using professional manpower, an early diagnosis of dementia and selection of a mild patient to be quickly and accurately made, and an even elderly subject who may not recognize text well to easily perform a checkup by presenting an image.

### Background Art

In general, dementia refers to a series of symptoms caused by brain diseases. When dementia progresses, the dementia affects thinking abilities, behaviors, and performance of daily life, and characteristics of the dementia refer to states in which abilities for daily activities lack due to declines in cognitive abilities.

In general, doctors diagnose dementia when two or more cognitive functions are significantly impaired. The cognitive functions include memory, language function, information understanding, spatial function, judgment, and attention. Dementia patients may have difficulty solving problems and controlling their emotions, and may experience personality changes.

Exact symptoms experienced by the dementia patients vary according to which part of the brain is damaged by diseases that cause dementia. Dementia occurs when some nerve cells in the brain stop functioning and lose connection with other cells and die, and the dementia usually progresses steadily. In other words, dementia gradually spreads to the brain and symptoms of patients worsen over time.

Dementia is a representative age-related neurodegenerative brain disease, with a prevalence of about 5 % to about 10 % in elderly people over 65 years of age worldwide. Most dementia patients show symptoms of progressive cognitive dysfunction, hallucinations, delusions, and loss of ability to live.

In the case of Alzheimer disease that is the most representative form of dementia, within nerve cells of the brain cortex, clumps of waste in which a protein called amyloid beta is coagulated are observed around nerve fiber bundles and brain cells, and the waste is estimated to cause necrosis of nerve cells.

Alzheimer disease described above is the most common disease that accounts for about 70 % of all dementia, and recently, about 600,000 Alzheimer disease patients are estimated to appear annually in Korea.

Alzheimer disease is a representative degenerative dementia disease that causes a decline in cognitive function and daily life through degeneration of cerebral nerve cells, is a scary disease that may even lead to death, and causes great pain not only to patients but also to the family members caring for the patients.

The diagnosis of dementia described above identifies dementia by identifying impairments in daily life and social activities due to cognitive impairment through detailed medical history taking and evaluation of symptoms and investigating cerebrovascular disease and brain atrophy by using brain imaging.

In the early stage of dementia, the dementia is not easily distinguished from senile forgetfulness, and thus is diagnosed by conducting a neuropsychological test that comprehensively evaluates not only a memory, but also a language ability, a calculation ability, a spatiotemporal perception ability, judgment, and the like. In order to diagnose this type of dementia, additional detailed tests need to be performed on the basis of various types of information obtained through interviews with patients/guardians and screening tests. The additional detailed tests include neuropsychological tests (SNSB), blood tests, various types of brain imaging tests (CT, MRI, PET), and the like.

As an example of the additional detailed test, magnetic resonance imaging (MRI) may play a significant role in distinguishing types of dementia. The above test may determine whether Alzheimer disease is close to dementia or vascular dementia and may also be of some help in determining whether or not Alzheimer disease is caused by another disease.

However, the prior art described above has the following drawbacks.

Dementia such as Alzheimer disease is an irreversible disease and no full cure has been developed therefor, and thus, a method of diagnosing dementia early and slowing progression thereof is the best method. Diagnoses of dementia through detailed history taking and evaluation by professional doctors are factors that prevent many patients from being quickly diagnosed due to a lack of professional manpower, while simultaneously increasing costs of selecting dementia patients among the entire population at a national level.

In addition, although existing brain imaging diagnoses used to diagnose dementia are expensive and precise equipment, brain diseases or brain atrophy may be identified only when in a very advanced state, and thus, the braining imaging diagnoses are not easy to be used for the purpose of early diagnoses of dementia.

In addition, dementia patients are usually elderly, illiterate, have low educational background, and may not recognize text well, and thus, tests using questionnaires and the like are not easy, a lot of time and manpower are needed for the tests.

Therefore, there is an urgent need to develop appropriate and groundbreaking technologies that may easily and quickly diagnose dementia at an early stage.

### Detailed Description of Disclosure

### Technical Problem

Accordingly, the present disclosure is provided to solve all the problems of the prior art as described above.

The present disclosure provides an image-based dementia diagnosis system and method which enable a dementia diagnosis to be quickly and conveniently performed without using professional manpower, an early diagnosis of dementia and selection of a mild patient to be quickly and accurately performed, and even an elderly subject who may not recognize text well to easily perform a checkup by presenting an image.

### Technical Solution to Problem

An image-based dementia diagnosis system according to the present disclosure includes: a text input device manipulated by a test subject to input text; a data storage device storing image data including different images drawn to represent one noun-type word as an image, storing data for memory interference, and storing dementia information for each test subject; a display configured to display diagnostic data and test data on a screen so that the test subject confirms the diagnostic data and the test data with eyes; a selection manipulator manipulated by the test subject to select selection items for the image data and data, which are displayed on the screen through the display; and a controller connected to and controlling the text input device, the data storage device, the display, and the selection manipulator, wherein the controller includes an information input unit into which the test subject inputs personal information through the text input device, an image presentation unit configured to extract 5 to 15 images from among images of the image data, sequentially output the extracted images on the screen of the display, and present the output images to the test subject, a memory interference unit 53 configured to output the data for memory interference on the screen of the display and interfere with a memory of the test subject, a test question generator configured to generate test questions by randomly mixing a plurality of images, which include the presented image as a correct answer and images other than the presented image as incorrect answers, after the output of the data for the memory interference is completed, a memory test performance unit configured to perform a memory test that enables the generated test questions to be sequentially output on the screen of the display, a correct answer to be selected when an image output as the test question through the selection manipulator is the same as the presented image, and an incorrect answer to be selected when not the same as the presented image, and a dementia determiner configured to determine whether or not dementia is present and/or a dementia status, according to a degree of incorrect answer rate of the test questions, which is confirmed through the memory test after the memory test is completed.

In addition, an image-based dementia diagnosis method according to the present disclosure includes: an information input operation of inputting personal information of a test subject into a personal information input field displayed to be input on the screen; an image provision operation of providing a plurality of different images drawn to represent one noun-type word as an image; an image presentation operation of extracting 5 to 15 images from among the provided images, and sequentially displaying and presenting the extracted images on the screen to allow the test subject to remember the images; a memory interference operation of interfering with a memory of the test subject by outputting, on the screen, data on which an attention of the test subject is concentrated after the presentation of the image is completed; a test question generation operation of generating test questions by randomly mixing a plurality of images, which include the presented image as a correct answer and images other than the presented image as incorrect answers, after the output of the data for the memory interference is completed; a memory test performance operation of performing a memory test that enables the generated test questions to be sequentially output, a correct answer to be selected when an image output as the test question is the same as the presented image, and an incorrect answer to be selected when not the same as the presented image; and a dementia determination operation of determining whether or not dementia is present and/or a dementia status, according to a degree of incorrect answer rate of the test questions, which is confirmed through the memory test, after the memory test is completed.

### Advantageous Effects of Disclosure

The present disclosure as described above enables a dementia diagnosis to be quickly and conveniently performed without using professional manpower, an early diagnosis of dementia and selection of a mild patient to be quickly and accurately performed, and even an elderly subject who may not recognize text well to easily perform a checkup by presenting an image. Accordingly, at the national level, screening of hundreds of thousands of dementia patients from among the entire population may be quickly and conveniently performed at a relatively low cost.

### Brief Description of Drawings

FIG. 1 is a schematic block diagram illustrating a dementia diagnosis system according to the present disclosure.
FIG. 2 is a schematic block diagram showing a controller of a dementia diagnosis system according to the present disclosure.
FIGS. 3 to 5 are example views illustrating an information input unit of a dementia diagnosis system according to the present disclosure.
FIGS. 6 to 8 are example views illustrating an image presentation unit of a dementia diagnosis system according to the present disclosure.
FIG. 9 is an example view illustrating various images used in a dementia diagnosis system according to the present disclosure.
FIGS. 10 to 15 are example views illustrating a memory interference unit of a dementia diagnosis system according to the present disclosure.
FIGS. 16 to 18 are example views illustrating a memory test performance unit of a dementia diagnosis system according to the present disclosure.
FIG. 19 is an example view illustrating various examples of a result display unit of a dementia diagnosis system according to the present disclosure.
FIG. 20 is a flowchart illustrating a dementia diagnosis method according to the present disclosure.

### Best Mode

The present disclosure relates to an image-based dementia diagnosis system and method which enable a dementia diagnosis to be quickly and conveniently performed without using professional manpower, an early diagnosis of dementia and selection of a mild patient to be quickly and accurately performed, and even an elderly subject who may not recognize text well to easily perform a checkup by presenting an image. The present dementia diagnosis system includes a text input device 10 through which a test subject may input text, a data storage device 20 that stores data for a diagnosis test, a display 30 that may display various types of images and data on a screen, a selection manipulator 40 that is manipulated by the test subject to select an item, and a controller 50 that is connected to various devices including the text input device 10 to control the devices and determines dementia by testing a memory on the basis of an image.

### Mode of Disclosure

Hereinafter, example embodiments of the present disclosure will be described in more detail with reference to the accompanying drawings. However, it should be understood that the present disclosure may be implemented in many different forms and is not limited to the described embodiments.

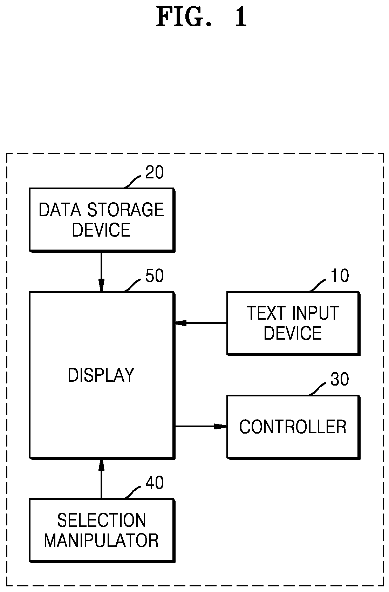

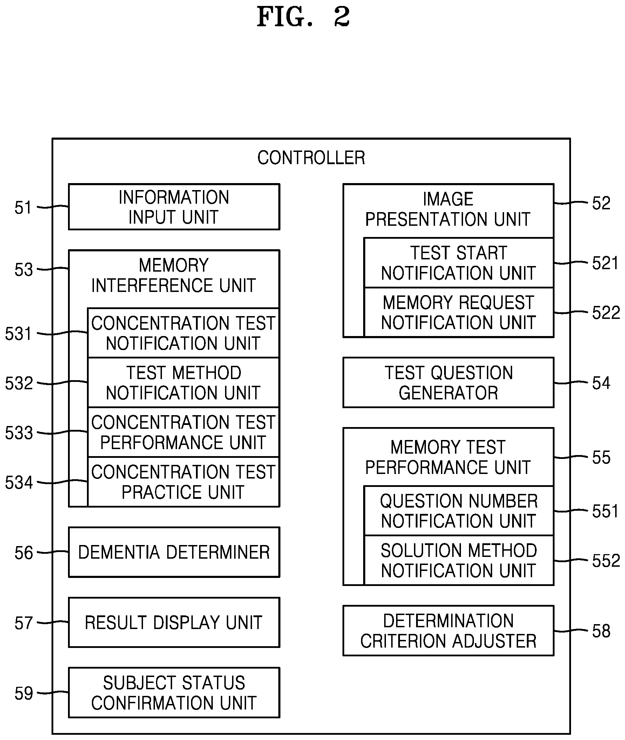

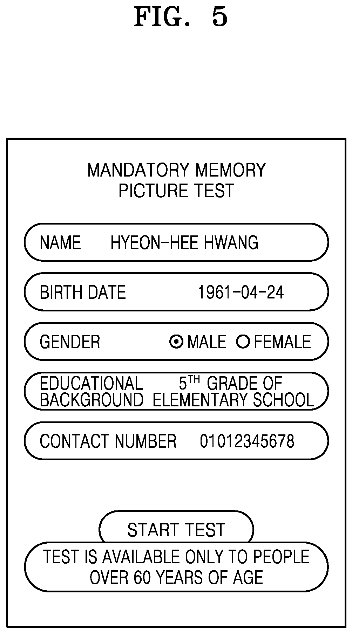

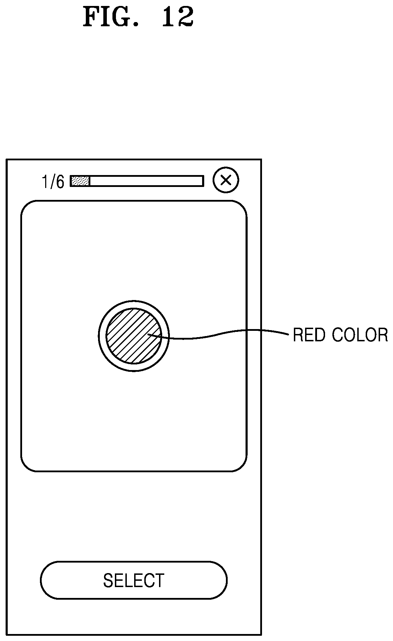

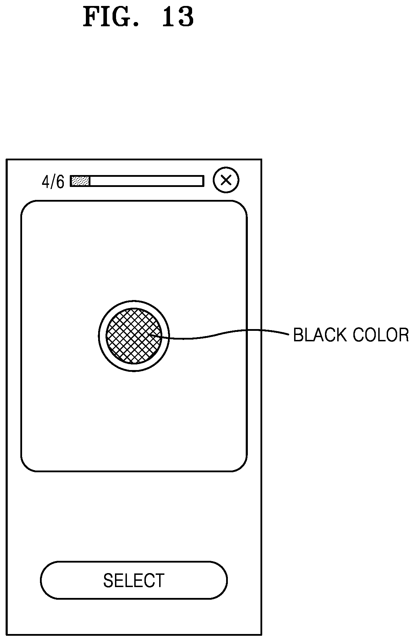

FIGS. 1 to 19 are views illustrating a dementia diagnosis system according to the present disclosure. As illustrated, an image-based dementia diagnosis system according to the present disclosure includes a text input device 10 through which a test subject may input text, a data storage device 20 that stores data for a diagnosis test, a display 30 that may display various types of images and data on a screen, a selection manipulator 40 that is manipulated by the test subject to select an item, and a controller 50 that is connected to various devices including the text input device 10 to control the devices and determines dementia by testing a memory on the basis of an image.

The text input device 10 is capable of inputting text like a keyboard and is manipulated by the test subject to input text.

The data storage device 20 is a storage device that may store digital data, such as a memory or a hard disk, may store image data including different images drawn to represent one noun-type word as an image, store data for memory interference, and store dementia information for each test subject.

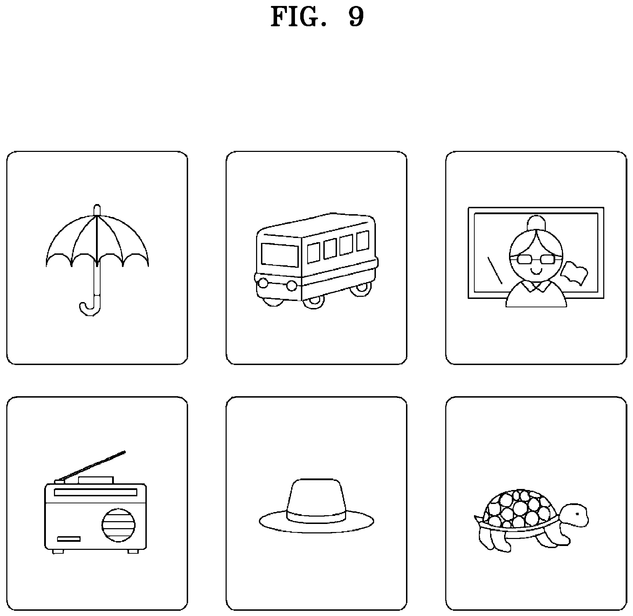

Here, the image is produced, converted into data, and stored for a memory test. The produced image is presented as an example in FIG. 9, and in the image, one noun-type word, for example, an umbrella, a bus, a teacher, a radio, a hat, a turtle, and the like, is represented as a picture one by one. In other words, the image is formed so that the test subject may see the image with eyes and associate one noun-type word.

The display 30 is a device that displays data or an image on the screen, such as a monitor or a liquid crystal display, and serves to display diagnostic data and test data on the screen so that the test subject may check the data with eyes.

The selection manipulator 40 is a device that is manipulated with a finger by the user to select an item or execute a command, such as a button or a touch screen, and is manipulated by the test subject to select selection items for image data and data displayed on the screen through the display 30.

The controller 50 is a device such as a microprocessor, is connected to the text input device 10, the data storage device 20, the display 30, and the selection manipulator 40, controls the same to present an image, interferes with a memory, and determines dementia by performing a memory test on the presented image.

The controller 50 as described above includes an information input unit 51, an image presentation unit 52, a memory interference unit 53, a test question generator 54, a memory test performance unit 55, and a dementia determiner 56.

The information input unit 51 enables the test subject to input personal information of the test subject through the text input device 10. The personal information of the test subject input as described above serves to distinguish test subjects from each other.

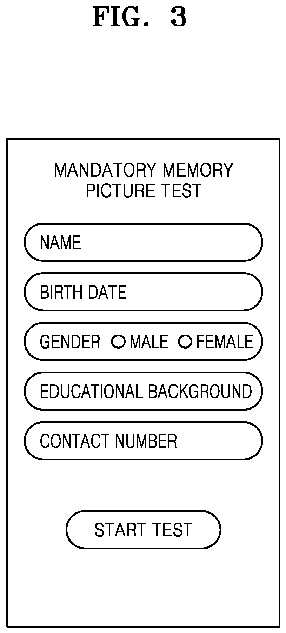

As shown in FIG. 3, the personal information is a name, a birth date, gender, an educational background, and a contact number of the test subject, and the items are input by the test subject.

Here, the birth date indicates the year, month, and day of birth of the test subject, the gender distinguishes a male and a female from each other, the educational background indicates the level of education of the test subject, and the contact number refers to a phone number with which the test subject may be contacted.

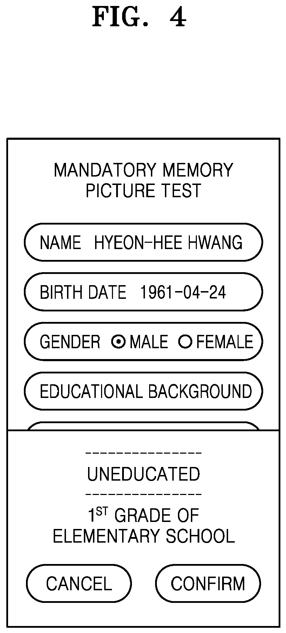

The educational background is allowed to be selected by grade, from uneducated to a graduate school to classify and identify educational background information in detail. As shown in FIG. 4, the educational background may be selected by scrolling through items defined by grade.

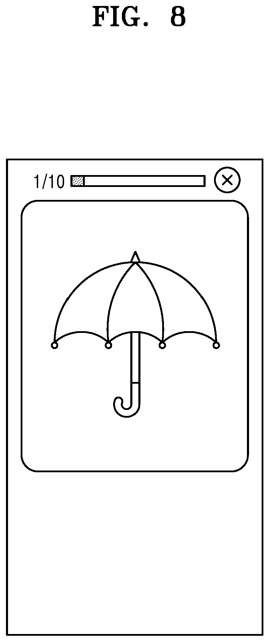

The image presentation unit 52 extracts 5 to 15 images from among images of image data, sequentially outputs the extracted images on the screen of the display 30, and presents the images to the test subject, so that the test subject may appropriately remember the images (refer to FIG. 8). As shown in FIG. 8, when an image is presented on the screen through the image presentation unit 52, a current state in which as the total number of images presented is the same as the number currently presented may displayed on the screen so that the test subject may confirm the current state with eyes.

The image presented through the image presentation unit 52 may be displayed on the screen for 1 to 3 seconds per one. When a time taken for displaying one image on the screen is shorter than 1 second, the test subject may not appropriately remember the image, and when the time taken for displaying one image on the screen is longer than 3 seconds, the test subject may easily remember the image, and thus, a discrimination ability may be reduced, such as a significant decrease in an incorrect answer rate.

The image presentation unit 52 as described above further includes a test start notification unit 521 that outputs, on the screen, a test start notification notifying a start of a test before presenting the image, and a memory request notification unit 522 that outputs, on the screen, a memory request notification requesting to remember the presented image while notifying the number of images to be presented after the notification of the test start is completed.

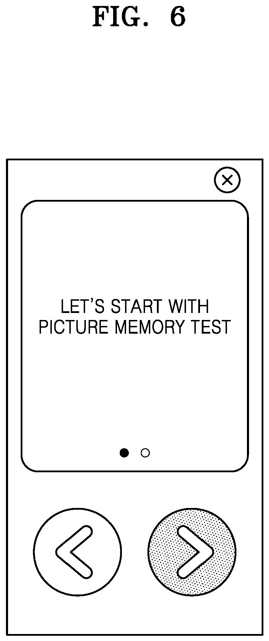

As presented as an example in FIG. 6, the test start notification unit 521 outputs a test start notification "A picture memory test will start." on the screen so that the test subject may recognize the same.

The test start notification as described above may be output not only as text but also as a voice through a voice output device such as a speaker connected to the controller 50, so that even a test subject who does not know text or have difficulty reading text may easily recognize the notification. The test start notification as described above may be output by selecting a screen output and a voice output or may be output by simultaneously performing the screen output and the voice output.

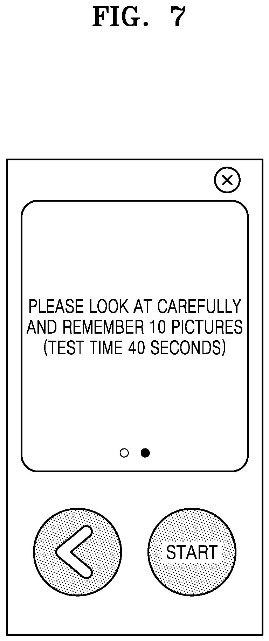

As presented as an example in FIG. 7, the memory request notification unit 522 outputs, on the screen, a memory request notification "Please, carefully look at and remember 10 pictures. (A test time 40 seconds)" to enable the test subject to recognize that the test subject needs to remember an image presented in the future. The memory request notification as described above may be output by connecting a speaker to the controller 50 and selecting a screen output and a voice output or simultaneously performing the screen output and the voice output.

After notifying the start of the test and notifying the memory request as described above, as shown in FIG. 8, extracted images are sequentially presented on the screen.

The memory interference unit 53 outputs the data for the memory interference on the screen of the display 30, and serves to concentrate attention of the test subject and interfere with a memory of the test subject for the image.

The memory interference unit 53 as described above includes a concentration test notification unit 531 that outputs, on the screen, a concentration test notification notifying a start of a concentration test to the test subject after the presentation of the extracted image is completed, a test method notification unit 532 that outputs, on the screen, a test method notification notifying the time taken for the test while notifying a concentration test method after the notification of the concentration test is completed, and a concentration test performance unit 533 that performs the concentration test after the notification of the test method is completed.

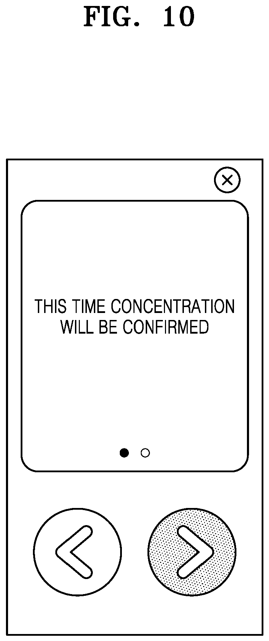

As presented as an example in FIG. 10, the concentration test notification unit 531 outputs a concentration test notification "This time, concentration will be confirmed" as text on the screen and induces a separate test to be performed to enable a memory interference process to start without the test subject recognizing that the memory interference process for the image is performed.

The concentration test notification as described above may be output by connecting a speaker to the controller 50 and selecting a screen output and a voice output or by simultaneously performing the screen output and the voice output.

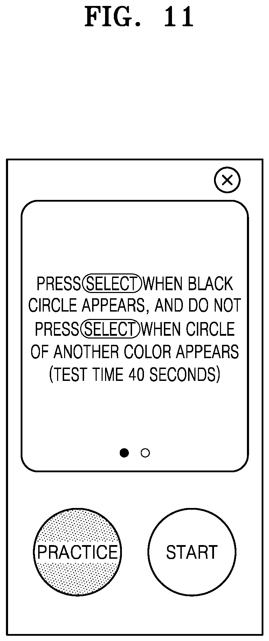

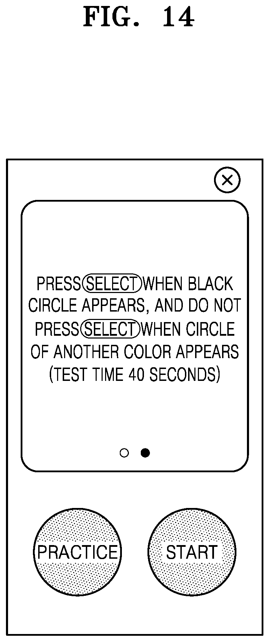

As presented as an example in FIGS. 11 and 14, the test method notification unit 532 outputs a test method notification "Press select when a black circle appears, but do not press select when a circle of a different color appears. (A test time 40 seconds)." as text on the screen, so that the test subject may appropriately recognize a method of performing the concentration test (which is actually the memory interference process).

The test method notification as described above may be output by connecting a speaker to the controller 50 and selecting a screen output and a voice output or simultaneously performing the screen output and the voice output.

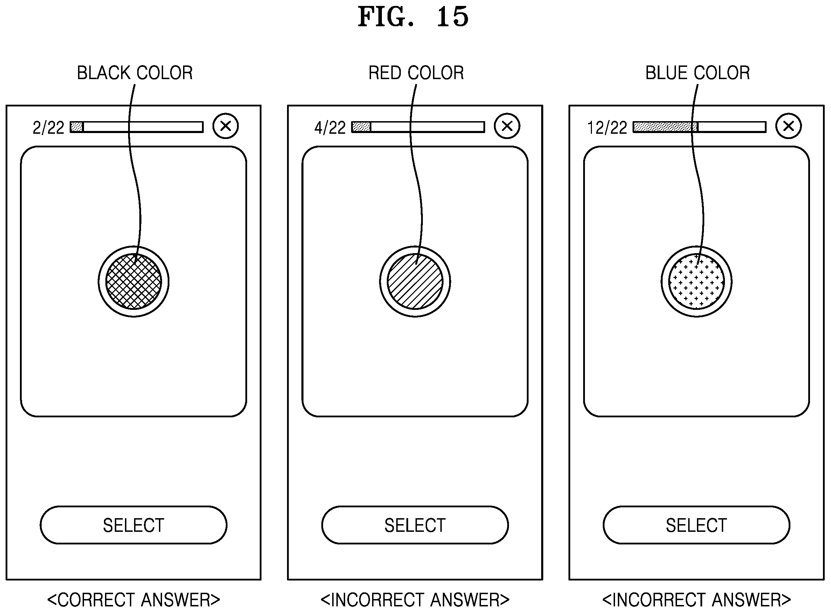

As presented as an example in FIG. 15, the concentration test performance unit 533 interrupts the test subject from remembering the image by performing the concentration test.

Here, as presented as an example in FIG. 15, the concentration test may be a color classification test in which several colors are sequentially presented and a particular color is selected as a correct answer and is to increase the concentration of attention of the test subject and easily perform a test for memory interference.

As shown in FIG. 15, the concentration test as described is a test including 10 to 30 questions using a black color as a correct answer and red and blue colors as incorrect answers and enables the attention of the test subject to be appropriately awakened and focus on the test by using the black color as the correct answer and the red and blue colors, which have a higher eye identification ability than the black color, as the incorrect answers. Here, the black, red, and blue colors may be displayed as circles of the same size to increase the concentration of the test subject.

An ordinary person may appropriately remember the image even when the memory interference is present, but a dementia patient may not appropriately remember the image due to memory disturbance, and thus, selection and discrimination of the dementia patient may be more accurately and easily made.

Here, the concentration test for memory interference may include 10 to 30 questions, and when the number of questions in the concentration test is less than 10, memory interference may not be appropriately performed, and when the number of questions in the concentration test is more than 30, excessive memory interference may be performed, and accordingly, an error may occur in a dementia determination.

As shown in FIG. 15, when questions are output on the screen through the concentration test performance unit 533, a current state in which the total number of questions output is the same as the number currently output may be displayed on the screen so that the test subject nay confirm the current state with eyes.

The memory interference unit 53 further includes a concentration test practice unit 534 that practices the concentration test by performing the concentration test 4 to 8 times in advance immediately before performing the concentration test. As shown as an example in FIGS. 11 to 13, the concentration test practice unit 534 increases the concentration of the test subject and increases a memory interference time to ensure that interference with a memory of the test subject is more reliable and effective.

The test question generator 54 serves to generate test questions by randomly mixing a plurality of images, which include the presented image as a correct answer and images other than the presented image as incorrect answers, after the output of the data for the memory interference is completed.

The test questions may be generated in 1.5 times to 2.5 times the number of images presented by the image presentation unit 52. For example, when the number of presented images is 10, the number of test questions may be generated as 20 (twice as many), and thus, 10 correct answer questions and 10 incorrect answer questions may be formed.

Accordingly, when the number of test questions is less than 1.5 times the number of presented images, a discrimination ability may be low and dementia may not be easily determined, and when the number of test questions is more than 2.5 times the number of presented images, the number of test questions is excessively high and an incorrect answer rate may be needlessly high.

The test questions may be OX-type questions in which "O" is selected for a correct answer and "X" is selected for an incorrect answer, so that the test subject may easily select the correct answer or the incorrect answer.

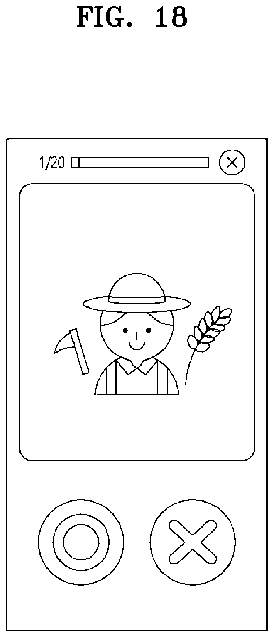

The memory test performance unit 55 serves to perform a memory test that enables the generated test questions to be sequentially output on the screen of the display 30, a correct answer to be selected when an image output as the test question through the selection manipulator 40 is the same as the presented image, and an incorrect answer to be selected when not the same as the presented image (refer to FIG. 18).

In other words, when the same image as the image presented by the image presentation unit 52 is presented on the screen through the memory test performance unit 55, the test subject selects "0" and displays the same as a correct answer. When the test subject does not remember the image presented through the image presentation unit 52 well, the test subject selects an incorrect answer, and the test subject is determined as a dementia patient when the same is repeated and an incorrect answer rate increases.

As shown in FIG. 18, when test questions are output on the screen through the memory test performance unit 55, a current state in which the total number of test questions output is the same as the number currently output is displayed on the screen to enable the test subject to identify the current state with eyes.

The memory test performance unit 55 as described above further includes a question number notification unit 551 that outputs, on the screen, a question number notification notifying the number of test questions to be output before outputting the test questions and a solution method notification unit 552 that outputs, on the screen, a solution method notification notifying the time taken for the test while notifying a question solution method after a notification of the number of question is completed.

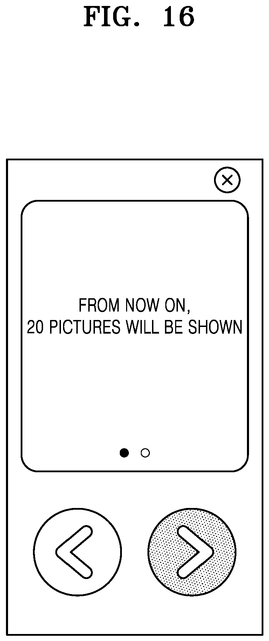

As presented as an example in FIG. 16, the question number notification unit 551 outputs a question number notification "From now on, 20 pictures will be shown" as text on the screen, so that the test subject may appropriately recognize the number of test question to be presented.

The question number notification as described above may be output by connecting a speaker to the controller 50 and selecting a screen output and a voice output or simultaneously performing the screen output and the voice output.

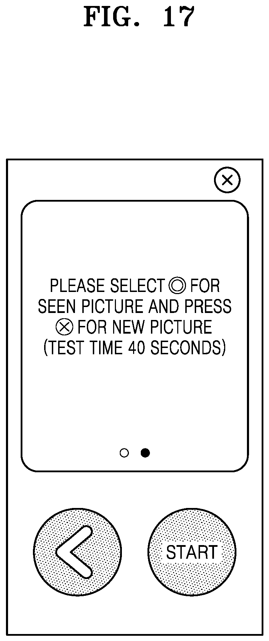

As presented as an example in FIG. 17, the solution method notification unit 552 outputs a solution method notification "Press O for a seen picture and X for a new picture. (A test time 40 seconds)" as text on the screen, so that the test subject may appropriately recognize a method of solving the test questions and a test time.

The solution method notification as described above may be output by connecting a speaker to the controller 50 and selecting a screen output and a voice output or simultaneously performing the screen output and the voice output.

The time taken for the test, which is displayed through the solution method notification unit 552, may be notified as 30 seconds to 50 seconds to notify the time taken for the memory test to the test subject in advance so that the test subject may appropriately prepare for the test.

The dementia determiner 56 serves to determine whether or not dementia is present and/or a dementia status, according to the degree of incorrect answer rate of test questions confirmed through the memory test after the memory test is completed. Here, the incorrect answer rate refers to a rate at which incorrect answers are selected from all test questions.

The controller 50 further includes a result display unit 57, a determination criterion adjuster 58, and a subject status confirmation unit 59.

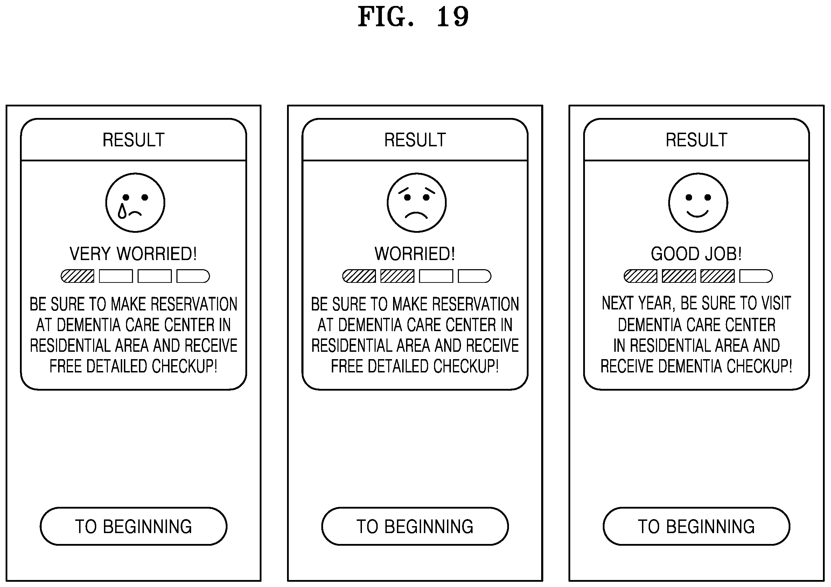

The result display unit 57 displays the results of diagnosis and test for the test subject on the screen of the display 30 after the dementia determination by the dementia determiner 56 is completed and, as shown in FIG. 19, serves to display the final result of the test on the screen to guide the test subject to a more precise dementia test while enabling the test subject to appropriately recognize a current state of the test subject.

For example, by evaluating 20 test questions and appropriately distinguishing between correct and incorrect answers, when the number of correct answers is 10 or less, the test result may be guided as "Very worried!", when the number of correct answers is 11 to 16, the test result may be guided as "Worried!", when the number of correct answers is 17 to 18, the test result may be guided as "Good job!", and when the number of correct answers is 18 to 20, the test result may be guided as "Perfect!".

The determination criterion adjuster 58 adjusts a determination criterion for determining whether or not dementia is present and/or a dementia status, according to a birth year, gender, and educational background of the test subject, and adjusts the dementia determination criterion according to the incorrect answer rate, according to the birth year, gender, and educational background of the test subject so that selection and determination of a dementia patient are made more accurately.

In other words, when the birth year (confirmed from the birth date that is input) of the test subject is earlier, the test subject is determined to be normal even at a relatively high incorrect answer rate, when the gender of the test subject is a female, the test subject has a higher probability of having dementia than a male of the same age and thus is determined to be a dementia patient even at a relatively low incorrect answer rate, and when the test subject has a lower educational background, the test subject has a high probability of having dementia and thus is determined to be a dementia patient even at a relatively low incorrect answer rate.

The adjustment of the determination criterion as described above is determined according to an incidence rate of dementia patients by age, gender, and educational background in each country or region. For example, by categorizing by age, gender, and education background, when the incidence rate of dementia patients is relatively high, the test subject is classified as a dementia patient even at a low incorrect answer. In contrast, by when categorizing by age, gender, and education background, when the incidence rate of dementia patients is relatively low the test subject is classified as a dementia patient only when the incorrect answer rate is significantly high.

As shown in FIG. 4, the subject status confirmation unit 59 compares the input birth date with a set age criterion to confirm, through an age restriction, whether or not the subject is a test subject and performs the test only when confirmed as the test subject, and designates only persons over a certain age as test subjects and excludes a person who does not need to be tested, so that a diagnostic test is performed more efficiently. For example, when the age restriction is set at 60 years old, the birth year of the test subject being input is compared with the age restriction, and a person younger than 60 years old is excluded from the test subject and accordingly, may not perform a dementia diagnostic test.

The present dementia diagnosis system may be configured as a mobile communication terminal such as a computer, an integrated dementia diagnostic device, or a smartphone in which the text input device 10, the data storage device 20, the display 30, the selection manipulator 40, and the controller 50 are modularized and integrated, and all functions may be produced as programs or applications, and loaded into the terminal and used.

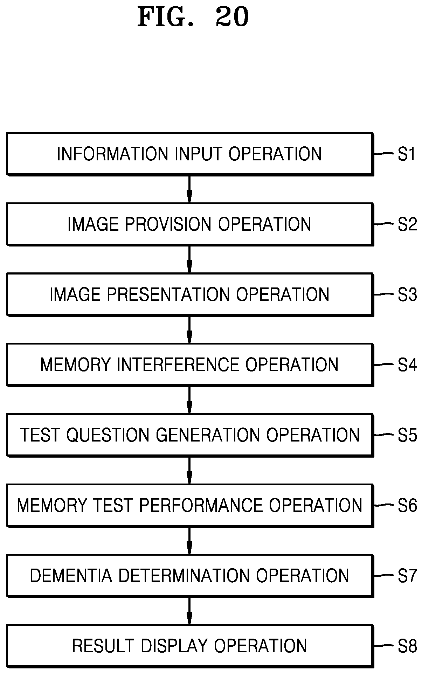

FIG. 20 is a flowchart illustrating a dementia diagnosis method according to the present disclosure.

As shown in FIG. 20, an image-based dementia diagnosis method according to the present disclosure includes an information input operation S1 of inputting personal information of a test subject on a screen, an image provision operation S2 of providing an image for a memory test, an image presentation operation S3 of extracting a portion from the provided image and presenting the extracted portion to the test subject on the screen, a memory interference operation S4 of interfering with a memory of the test subject for the image, a test question generation operation S5 of generating test questions for the memory test, a memory test performance operation S6 of outputting the test questions to the test subject and performing the memory test, a dementia determination operation S7 of determining whether or not dementia is present, on the basis of an incorrect answer rate confirmed through the memory test, and a result display operation S8 of displaying the result of the test for the test subject on the screen, which is confirmed through the dementia determination, and dementia is diagnosed by sequentially performing the operations as described above.

Subsequent to the dementia determination operation S7, the image-based dementia diagnosis method may further include the result display operation S8 of displaying the results of diagnosis and test for the test subject on the screen after the dementia determination is completed.

In the information input operation S1, the personal information is a name, a birth date, gender, an educational background of the test subject, and a contact number, and the educational background may be set to be selected by grade from uneducated to a graduate school.

The dementia determination operation S7 may further include a determination criterion adjustment process of adjusting a determination criterion for determining whether or not the dementia is present and/or a dementia status, according to the birth year, gender, and educational background of the test subject.

The information input operation S1 may further include a subject status confirmation process of comparing the input birth date with a set age criterion to confirm, through age restriction, whether or not a subject is a test subject and performing the test only when confirmed as the test subject.

In the image presentation operation S3, the image may be displayed on the screen for 1 seconds to 3 seconds per one image.

The image presentation operation S3 may further include a test start notification process of outputting, as text and/or voice, a test start notification notifying a start of the test before presenting the image, and a memory request notification process of outputting, as text and/or voice, a memory request notification requesting to remember the presented image while notifying the number of images to be presented after the test start notification process is completed.

In the test question generation operation S5, the number of test questions may be 1.5 times to 2.5 times the number of images presented in the image presentation operation S3.

In the test question generation operation S5, the test questions may be OX-type questions in which O is selected for a correct answer and X is selected for an incorrect answer.

The memory test performance operation S6 may further include a question number notification process of outputting, as text and/or voice, a question number notification notifying the number of test questions to be output before outputting the test questions and a solution method notification process of outputting, as text and/or voice, a solution method notification notifying the time taken for the test while notifying a question solution method after the question number notification process is completed.

In the solution method notification process, the time taken for the test may be notified as 30 seconds to 50 seconds.

The memory interference operation S4 may include a concentration test notification process of outputting, as text and/or voice, a concentration test notification notifying a start of a concentration test to the test subject after the presentation of the extracted image is completed, a test method notification process of outputting, as text and/or voice, a test method notification notifying the time taken for the test while notifying a concentration test method after the concentration test notification process is completed, and a concentration test performance process of performing the concentration test after the test method notification process is completed.

In the memory interference operation S4, the concentration test may be a color classification test in which several colors are sequentially presented and a particular color is selected as a correct answer.

The concentration test is a test including 10 to 30 questions which use a black color as a correct answer and red and blue colors as incorrect answers, and the black, red, and blue colors may be displayed as circles of the same size.

The memory interference operation S4 may further include a concentration test practice process of practicing the concentration test by performing the concentration test 4 times to 8 times in advance immediately before performing the concentration test performance process.

The present dementia diagnosis system constructed as described above is a groundbreaking invention that presents an image to a test subject, performs a memory test on the image in a state in which the presented image is not well remembered due to memory interference, and thus enables dementia to be rapidly and accurately selected.

Embodiments according to the present disclosure may be implemented in the form of a computer program that may be executed through various components on a computer, and the computer program may be recorded on a computer-readable medium. Here, the medium may include magnetic media, such as a hard disk, a floppy disk, and a magnetic tape, optical recording media, such as CD-ROM and DVD, magneto-optical media, such as a floptical disk, and hardware devices, such as ROM, RAM, and flash memory devices specially configured to store and execute program instructions.

Meanwhile, the computer program may be specially designed and configured for the present disclosure, or may be known to and used by those skilled in the art of the computer software field. Examples of the computer program may include not only machine language code generated by a compiler but also high-level language code that may be executed by a computer by using an interpreter or the like.

According to one embodiment, the method according to various embodiments of the present disclosure may be included and provided in computer program products. The computer program products may be traded between sellers and buyers as commodities. The computer program products may be distributed in the form of device-readable storage media (e.g., compact disc read only memory (CD-ROM)), or may be distributed (e.g., downloaded or uploaded) online via an application store (e.g., Play Store TM) or between two user devices. When distributed online, at least a portion of a computer program product may be temporarily stored or temporarily generated in a device-readable storage medium such as a server of a manufacturer, a server of an application store, or a memory of a relay server.

The operations constituting the method according to the present disclosure may be performed in any appropriate order unless an order of the operations is explicitly stated or stated to the contrary. The present disclosure is not necessarily limited according to the order of description of the operations. The use of all examples or example terms (e.g., and the like) in the present disclosure is simply to describe the present disclosure in detail, and the scope of the present disclosure is limited due to the examples or example terms unless limited by claims. In addition, those skilled in the art may appreciate that various modifications, combinations and changes may be made according to design conditions and factors within the scope of the appended claims or equivalents thereof.

Meanwhile, in the description of the present disclosure, particular embodiments have been described, but it may be obvious to one of ordinary skill in the art that various modifications may be made without departing from the scope of the present disclosure.

### Itemized list of embodiments

Item 1. An image-based dementia diagnosis system comprising:
a text input device (10) manipulated by a test subject to input text;
a data storage device (20) storing image data comprising different images drawn to represent one noun-type word as an image, storing data for memory interference, and storing dementia information for each test subject;
a display (30) configured to display diagnostic data and test data on a screen so that the test subject confirms diagnostic data and test data with eyes;
a selection manipulator (40) manipulated by the test subject to select selection items for the image data and data, which are displayed on the screen through the display (30); and
a controller (50) connected to and controlling the text input device (10), the data storage device (20), the display (30), and the selection manipulator (40), wherein the controller (50) comprises:
   an information input unit (51) into which the test subject inputs personal information through the text input device (10);
   an image presentation unit (52) configured to extract (5) to (15) images from among images of the image data, sequentially output the extracted images on the screen of the display (30), and present the output images to the test subject;
   a memory interference unit (53) configured to output the data for memory interference on the screen of the display (30) and interfere with a memory of the test subject;
   a test question generator (54) configured to generate test questions by randomly mixing a plurality of images, which include the presented image as a correct answer and images other than the presented image as incorrect answers, after the output of the data for the memory interference is completed;
   a memory test performance unit (55) configured to perform a memory test that enables the generated test questions to sequentially output on the screen of the display (30), a correct answer to be selected when an image output as the test question through the selection manipulator (40) is the same as the presented image, and an incorrect answer to be selected when not the same as the presented image; and
   a dementia determiner (56) configured to determine whether or not dementia is present and/or a dementia status, according to a degree of incorrect answer rate of the test questions, which is confirmed through the memory test after the memory test is completed.

Item 2. The image-based dementia diagnosis system of item 1, wherein the controller (50) further comprises a result display unit (57) configured to display results of diagnosis and test for the test subject on the screen of the display (30) after the dementia determination by the dementia determiner (56) is completed.

Item 3. The image-based dementia diagnosis system of item 1, wherein the personal information into the information input unit (51) is a name, a birth date, gender, an educational background, and a contact number of the test subject, and the educational background is selected by grade, from uneducated to a graduate school.

Item 4. The image-based dementia diagnosis system of item 3, wherein the controller (50) further comprises a determination criterion adjuster (58) configured to adjust a determination criterion for determining whether or not the dementia is present and/or the dementia status, according to a birth year, gender, and the education background of the test subject.

Item 5. The image-based dementia diagnosis system of item 3, wherein the controller (50) further comprises a subject status confirmation unit (59) configured to compare the input birth date with a set age criterion to confirm, through an age restriction, whether or not corresponding to the test subject and perform the test only when confirmed as a test subject.

Item 6. The image-based dementia diagnosis system of item 1, wherein the image presentation unit (52) further comprises:
a test start notification unit (521) configured to output, on the screen, a test start notification notifying a start of the test before presenting the image; and
a memory request notification unit (522) configured to output, on the screen, a memory request notification requesting to remember the presented image while notifying a number of images to be presented after the notification of the test start is completed.

Item 7. The image-based dementia diagnosis system of item 1, wherein the test question generator (54) is configured to generate test questions of 1.5 times to 2.5 times a number of images presented by the image presentation unit (52).

Item 8. The image-based dementia diagnosis system of item 1, wherein the memory test performance unit (55) further comprises:
a question number notification unit (551) configured to output, on the screen, a question number notification notifying a number of test questions to be output before outputting the test questions; and
a solution method notification unit (552) configured to output, on the screen, a solution method notification notifying a time taken for the test while notifying a question solution method after the notification of the number of questions is completed.

Item 9. The image-based dementia diagnosis system of item 1, wherein the memory interference unit (53) comprises:
a concentration test notification unit (531) configured to output, on the screen, a concentration test notification notifying a start of a concentration test to the test subject after the presentation of the extracted image is completed;
a test method notification unit (532) configured to output, on the screen, a test method notification notifying a time taken for the test while notifying a concentration test method after the notification of the concentration test is completed; and
a concentration test performance unit (533) configured to perform the concentration test after the notification of the test method is completed.

Item 10. The image-based dementia diagnosis system of item 9, wherein the concentration test is a color classification test in which several colors are sequentially presented and a particular color is selected as a correct answer.

Item 11. The image-based dementia diagnosis system of item 10, wherein the concentration test is a test comprising 10 to 30 questions which use a black color as a correct answer and red and blue colors as incorrect answers, and the black, red, and blue colors are displayed as circles of a same size.

Item 12. The image-based dementia diagnosis system of item 11, wherein the memory interference unit (53) further comprises a concentration test practice unit (534) configured to practice the concentration test by performing the concentration test 4 times to 8 times in advance immediately before performing the concentration test.

Item 13. An image-based dementia diagnosis method comprising:
an information input operation (S1) of inputting personal information of a test subject into a personal information input field displayed to be input on the screen;
an image provision operation (S2) of providing a plurality of different images drawn to represent one noun-type word as an image;
an image presentation operation (S3) of extracting 5 to 15 images from among the provided images, and sequentially displaying and presenting the extracted images on the screen to allow the test subject to remember the images;
a memory interference operation (S4) of interfering with a memory of the test subject by outputting, on the screen, data on which an attention of the test subject is concentrated after the presentation of the image is completed;
a test question generation operation (S5) of generating test questions by randomly mixing a plurality of images, which include the presented image as a correct answer and images other than the presented image as incorrect answers, after the output of the data for the memory interference is completed;
a memory test performance operation (S6) of performing a memory test that enables the generated test questions to be sequentially output, a correct answer to be selected when an image output as the test question is the same as the presented image, and an incorrect answer to be selected when not the same as the presented image; and
a dementia determination operation (S7) of determining whether or not dementia is present and/or a dementia status, according to a degree of incorrect answer rate of the test questions, which is confirmed through the memory test, after the memory test is completed.

Item 14. A computer-readable recording medium having recorded thereon a program for causing a computer to execute the method of claim item 13.

## Claims

1. A system for diagnosing dementia through image presentation, the system comprising:
a controller (50) configured to control the system, wherein the controller (50) is configured to:
present a predetermined image to a test subject;
after completion of the image presentation, generate a memory test by randomly mixing a plurality of images, including the presented image as a correct answer and other images as incorrect answers, and output the memory test;
perform the memory test by allowing the test subject to select the correct answer if the presented image matches the image in the memory test, or to select an incorrect answer otherwise;
determine whether dementia is present and/or a dementia status based on the degree of incorrect answer rate identified through the memory test after the memory test is completed; and
after the presentation of the image and before perform the memory test, execute a concentration test that outputs material capable of focusing the test subject's attention to interfere with the subject's memory of the image, wherein the concentration test comprises:
outputting a concentration test start notification indicating the beginning of the concentration test to the test subject,
outputting a concentration test method notification indicating how the concentration test is to be performed, and
performing the concentration test after the output of the test method notification is completed.

2. The system of claim 1, wherein the controller (50) is further configured to display a diagnostic result and test result of the test subject after determining whether dementia is present and/or the dementia status.

3. The system of claim 1, wherein the controller (50) is further configured to adjust a determination criterion for dementia based on at least one of the test subject's birth year, gender, or educational background.

4. The system of claim 1, wherein the controller (50) is further configured to output a test start notification on a screen (30) before presenting the image, and after the test start notification is output, to output a memory request notification requesting the subject to memorize the presented image.

5. The system of claim 1, wherein the controller (50) is further configured to output a solution method notification indicating how to solve the memory test before the memory test is output.

6. The system of claim 1, wherein the controller (50) is further configured to perform the concentration test comprising a color classification test in which several colors are sequentially presented and a specific color is selected as a correct answer.

7. The system of claim 1, wherein the controller (50) is further configured to perform a concentration test exercise prior to the actual concentration test to allow the test subject to practice.

8. A computer implemented method for diagnosing dementia, the method comprising:
presenting a predetermined image to a test subject;
after completion of the image presentation, generating a memory test by randomly mixing a plurality of images, including the presented image as a correct answer and other images as incorrect answers, and outputting the memory test;
performing the memory test by allowing the test subject to select the correct answer if the presented image matches the image in the memory test, or to select an incorrect answer otherwise;
determining whether dementia is present and/or a dementia status based on the degree of incorrect answer rate identified through the memory test after the memory test is completed;
after the image presentation step and before the step of performing the memory test, executing a concentration test that outputs material capable of focusing the test subject's attention to interfere with the subject's memory of the image, wherein the concentration test comprises:
outputting a concentration test start notification indicating the beginning of the concentration test to the test subject,
outputting a concentration test method notification indicating how the concentration test is to be performed, and
performing the concentration test after the output of the test method notification is completed.

9. A computer program comprising instructions which, when the program is executed by an electronic device having processing capabilities, cause the electronic device to carry out method of claim 8.

10. A computer-readable recording medium having recorded thereon a computer program according to claim 9.
